# EUROPEAN PATENT SPECIFICATION

(11) **EP 2 380 577 B1**
(45) Date of publication and mention of the grant of the patent: **13.11.2013**
(21) Application number: 10382090.8
(22) Date of filing: 21.04.2010
(51) Int. Cl.: A61K 8/19, A61K 8/27, A61K 33/00, A61K 33/30, A61Q 17/00

(54) **Anti-jellyfish combinations**
Kombinationen gegen Quallen
Combinaisons contre les méduses

(43) Date of publication of application: 26.10.2011
(73) Proprietor: Antonio Puig, S.A., 08021 Barcelona (ES)
(72) Inventor: Ginestar González, José, 08030, Barcelona (ES); Panyella Costa, David, 08030, Barcelona (ES); Catalá Escursell, Remei, 08030, Barcelona (ES); Vidal Tasa, Jordi, 08030, Barcelona (ES); Montanyà Dejuan , Sònia, 08030, Barcelona (ES)
(74) Representative: ZBM Patents

(56) References cited:
- WO-A1-98/53807
- WO-A1-03/096993
- WO-A2-2005/076731
- EDWARDS ET AL: "Portuguese Man-of-war (Physalia physalis) venom induces calcium influx into cells by permeabilizing plasma membranes" CA, 1 January 2000 (2000-01-01), XP002335194
- SANTORO G ET AL: "THE DISCHARGE OF IN-SITU NEMATOCYSTS OF THE ACONTIA OF AIPTASIA-MUTABILIS IS A CALCIUM ION-INDUCED RESPONSE" JOURNAL OF EXPERIMENTAL BIOLOGY, vol. 156, 1991, pages 173-186, XP9135945 ISSN: 0022-0949
- HEEGER T ET AL: "Protection of human skin against jellyfish (Cyanea capillata) stings" MARINE BIOLOGY, BERLIN, DE LNKD- DOI:10.1007/BF00349710, vol. 4, no. 113, 1 January 1992 (1992-01-01), pages 669-678, XP002076620 ISSN: 0025-3162

## Description

The present invention relates to the use of combinations for avoiding the sting of venomous organisms from aquatic environments. Particularly, the invention relates to the use of combinations of lanthanum and zinc cations for avoiding the sting of cnidarians and myxozoans.

### BACKGROUND ART

Jellyfish stings are a common occurrence among divers and beach-lovers worldwide with an estimated 150 million envenomations annually. Fatalities and hospitalizations occur frecuently in the Indo-Pacific regions, while fatal stings are unusual in Europe. Despite the lower mortality, jellyfish envenomation is nevertheless an over-all concern in European beaches, specially for children.

In addition to jellyfish, other animals of the same group, the phylum Cnidaria, are toxic to humans to greater or lesser effect. The Cnidaria phylum includes animals like sea anemones, sea wasps, corals and box jellyfish. All of these animals posses a type of venomous cells, called cnidocytes, that they use mainly for capturing prey. These cells are responsible for the stinging of Cnidaria.

Each cnidocyte cell contains a organelle called a cnidocyst, which comprises a bulb-shape capsule containing a coiled hollow thread-like structure attached to it. The externally-oriented side of the cell also has a hair-like trigger called a cnidocil. The cnidocyst capsule stores a large concentration of calcium ions. When the trigger is activated by certain stimuli, the calcium ions are released from the capsule into the cytoplasm of the cnidocyte causing a large concentration gradient of calcium across the cnidocyte plasma membrane. The resulting osmotic pressure causes a rapid influx of water into the cell. This increase in water volume in the cytoplasm forces the coiled cnidocyst to eject rapidly penetrating the target organism, and the hollow thread-like structure is everted into it. This discharge takes no more than a few microseconds, and is said to be one of the most rapid mechanical events found in nature. After penetration, the toxic content of the cnidocyst is injected into the target organism.

In order to regulate discharge, cnidocytes are connected as "batteries", containing several types of cnidocytes connected to supporting cells and neurons. The supporting cells contain chemoreceptors, which, together with the mechanoreceptor on the cnidocyte (cnidocil), allow only the right combination of stimuli to cause discharge, such as prey swimming, and chemicals found in prey cuticle or skin.

Parasitic animals of the group of the myxozoans posses organells that highly resemble cnidocysts. In the case of myxozoans, these organells are named polar capsules and play a major role in the infectious cycle of these aquatic parasites.

Post-stinging remedies such as soothing creams and antidotes are usually applied in the treatment of cnidarian envenomation. However, the effectiveness of these remedies are often unsatisfactory so that the best practice is to avoid contact with these animals. Rash guards and stinger suits are excellent in preventing contact with cnidarias and are recommended for divers in regions where lethal jellyfish are common, but they are not practical for bathers, nor are they completely effective when dealing with myxozoan infection. Therefore, other prevention strategies are desirable to avoid the sting of cnidarians and the infection by myxozoans.

In this direction, Salleo et al (J. exp. Biol., 1994, vol. 187, p. 201-206) disclosed that Gd³⁺ and La³⁺ could be useful in preventing stings from harmful Cnidaria. These authors investigated the activation properties of in situ cnidocytes of Pelagia noctiluca by physical contact with a gelatin probe that, besides stimulating the cnidocyte battery, retained the discharged cnidocysts, thereby allowing a quantitative evaluation of the response. In oral arms previously treated with 2 mmol/L La³⁺ or 20 µmol/L Gd³⁺, the discharge was inhibited. This results confirmed the Ca²⁺-dependence of cnidocyte activation and indicated that these cations could be useful to inhibit discharge.

Additionaly, WO 98/53807 discloses compositions comprising an effective amount of antihistamines and/or an effective amount of at least one metal cation for inhibiting cnidocyst or polar capsule discharge. The preferred metal cations for this use include Ca, Mg, Na, Mn, Co, K and Fe. In a further patent application, WO 05/076731, the same author discloses compositions comprising an oil in water emulsion containing an inorganic salt, wherein said salt comprises Ca, Mg, Na, K, Zn, Ti, Ga or La cation for the same use. Preferred compositions are those containing Ca, Mg and La cations. The provided emulsions are to be used in the prevention of cnidocyst discharge.

In a collaborative study, the protective effects of a composition containing Ca and Mg cations formulated in a sunscreen lotion was evaluated against Chrysaora fuscescens and Chiropsalmus quadrumanus jellyfish (Boer Kimball A. et al, Wilderness and Environmental Medicine, 2004, vol. 15, p. 102-108). The study concluded that the jellyfish sting inhibitor does not eliminate the sting from C. fuscescens or C. quadrumanus jellyfish, but significantly reduces the frequency and severity of stings, thus providing some positive effects.

Thus, further strategies are needed to minimise the effects of cnidarian envenomation and provide practical protection for recreational and professional swimmers. The provision of compositions useful for inhibiting the discharge of cnidocysts, thereby avoiding the sting of cnidarians and the infection by myxozoans, is still of great interest in the fields of cosmetics and pharmacy.

### SUMMARY OF THE INVENTION

It has been surprisingly found that the combination of zinc and lanthanum cations is highly effective for inhibiting cnidocyst discharge.

As will be shown in the examples below, the combination of zinc and lanthanum cations has a synergistic interaction in the inhibition of cnidocyst discharge, without any antagonistic effects. Thus, this combination improves the effects of previous anti-cnidarian compositions by providing an enhanced inhibition of cnidocyst discharge. In other words, in the presence of lanthanum and zinc cations, the cnidocytes of the cnidarian or myxozoan organism, even though contacting with a foreign body, do not recognise it as danger or nourishment, with the result that its stinging organells are not triggered. The provided combination exhibits an enhanced effect in avoiding the sting of poisonous organisms that contain cnidocysts.

Thus, a first aspect of the present invention is the use of a combination of an effective amount of Zn²⁺ and La³⁺ cations for avoiding the sting of poisonous organisms that contain cnidocysts.

The combinations of the invention can be incorporated into topical compositions, and formulated in such a way as to provide a physical barrier over the skin that prevents that natural skin stimuli reach the cnidarian to trigger the mechanism of cnidocyst discharge. As well as exerting a barrier effect, the present compositions contain an effective amount of zinc and lanthanum cations as active agents that inhibit cnidocyst discharge. The inhibitory activity of the synergistic combination, together with the barrier effect of the topical formulation, is greatly effective in avoiding the sting of cnidarians.

Thus, a second aspect of the invention provides the use of a topical composition comprising an effective amount of Zn²⁺ and La³⁺ cations, together with appropriate topical pharmaceutically or cosmetically acceptable excipients and/or carriers, for avoiding the sting of poisonous organisms that contain cnidocysts.

Besides being effective in avoiding the sting of cnidarian organisms, the combination of the invention also has the ability of enhancing cutaneous integrity. It also has an effect in the relief of minor skin irritations and in protection against microbial infections due to its properties as mild astringent and as topical biocide.

In a third aspect, the invention provides a method for avoiding the sting of an organism selected from the group consisting of Cnidaria and Myxozoa comprinsing the topical administration of the composition as defined above, prior to contact with said organism.

### DETAILED DESCRIPTION OF THE INVENTION

Cnidaria is a phylum containing over 9000 species of animals found exclusively in aquatic and mostly marine environments. Some cnidarians are well known by their common name, for instance, jellyfish, sea anemones, sea wasps, corals and box jellyfish. Their distinguishing feature is cnidocytes, specialized stinging cells they use mainly for capturing prey.

A cnidocyte (also called nematocyte or cnidoblast) is a type of venomous cell unique to the phylum Cnidaria. These cells contain a stinging structure called cnidocysts, as well as an externally-orientated hair-like trigger called a cnidocil.

The term "cnidocyst" (also called cnida or nematocyst) is defined as the intracellular, stinging, capsule-like structure characteristic of all cnidarians. When triggered, the cnidocyst is rapidly ejected penetrating a target organism and everting a hollow thread-like structure which injects neurotoxins into it. This mechanism is responsible for the sting delivered by certain cnidarias, such as jellyfish.

Myxozoans are parasitic animals of aquatic environments. Infection occurs through valved spores that contain one or two sporoblast cells and one or more polar capsules that contain filaments which anchor the spore to its host. The sporoblasts are then released as a motile form, called an amoebula, which penetrates the host tissues. Phylogenetical studies, as well as the great similarity between myxozoan polar capsules and cnidarian cnidocysts, have driven many experts to consider myxozoans as severely modified members of the phylum Cnidaria. In the sense of the present invention, the term "cnidocyst" will be used to refer both to cnidarian cnidocysts and myxozoan polar capsules.

The term "cation source" refers to any substance which is capable of supplying a positively charged ion, such as a salt. Any salt of the cation may be used in the present invention.

As mentioned above, the present inventors have surprisingly found that the combination of zinc and lanthanum cations has a synergistic interaction in the inhibition of cnidocyst discharge, thus exhibiting an enhanced activity in avoiding the sting of organsims than contain such cnidocysts.

In a preferred embodiment of the first aspect of the invention, the cnidocysts to be inhibited belong to an organism selected from the group consisting of Cnidaria and Myxozoa. Preferably, the combinations of the invention are used to avoid the sting of jellyfish.

Although no uniformity about the calculations and definitions of interactions exists, the most accepted description defines an interaction when the observed effect of the combination differs with the effects of the individual constituents. The interaction study of two substances involve both, the individual efficacy evaluation and the effects of different combinations.

The graphical representation of the interaction between two active compounds could be expressed as follows. The x-axis shows the dose pair's combinations and the individual substance concentrations tested in the study, and in the y-axis the results obtained expressed as number of discharged nematocysts per mm². The straight line connecting the effects of both substances on their own is the locus of points that will produce this effect in a simple additive combination, the additivity line. A result below the line of additivity suggests a synergism and a result over the line an antagonism.

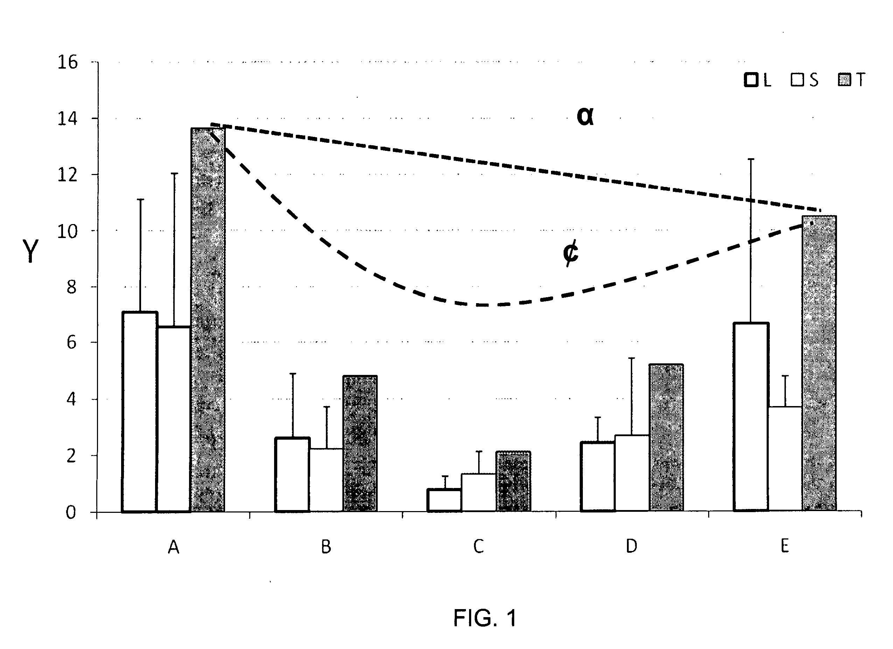

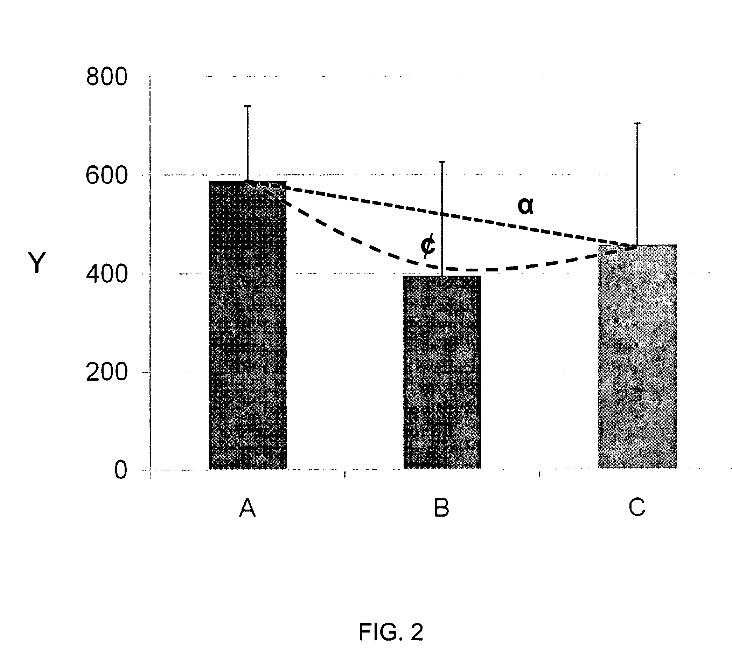

The graphical representation of the interaction between lanthanum and zinc cations is shown in FIG. 1 and FIG. 2. As can be observed, the combination of these agents yields a synergistic effect as shown by the inhibition of the discharge of cnidocysts from Pelagia noctiluca and Rhizostoma pulmo.

The ratio of zinc to lanthanum cations to be used for the combinations of the invention is comprised between 1:100 and 100:1 (Zn²⁺: La³⁺). Preferably the ratio of Zn²⁺ to La³⁺ is comprised between 1:50 and 50:1, more preferably between 1:20 and 20:1, still more preferably between 1:10 and 10:1, and even still more preferably, the ratio of Zn²⁺ to La³⁺ is comprised between 1:6 and 6:1. In a preferred embodiment the combination of the invention comprises lantanum and zinc cations in a ratio of 6:1.

The cations of the provided combinations can be in the form of any suitable cation source, such as a salt. In a particular embodiment, the cation source is a zinc halide and a lanthanum halide. Preferably, the cation source is zinc chloride and lanthanum chloride.

The combinations described above can be incorporated into topical compositions to be applied to the skin of a subject to protect said subject against the stinging of organisms of the cnidarian group and the infection by myxozoans.

In one embodiment, an effective amount of the combination in the sense of the present invention is comprised between 0.01 and 4 % of Zn²⁺ and between 0.01 and 4 % of La³⁺. Concentrations are expressed in percentage by weight (% w/w) of active agents, i.e. cations in the total composition. In another embodiment, the amount of each cation, zinc and lanthanum, is comprised between 0.05 and 2.5 %, preferably between 0.1 and 2 % of the total composition. In a preferred embodiment, the amount of Zn²⁺ is 0.07 % and the amount of La³⁺ is 0.43 % of the total composition.

The topical composition of the invention is formulated preferably as an emulsion. An emulsion is a dispersed system comprising at least two immiscible phases, one phase dispersed in the other as droplets. An emulsifying agent is typically included to improve stability. When water is the dispersed phase and an oil is the dispersion medium, the emulsion is termed a water-in-oil emulsion (w/o). When an oil is dispersed as droplets throughout the aqueous phase, the emulsion is termed an oil-in-water emulsion (o/w). Other types of emulsions known in the art are multiple emulsions, such as water-in-oil-in-water emulsions (w/o/w), GELTRAP emulsions, where the aqueous intern phase is gelified and it is covered by the oil phase, and SWOP emulsions, also known as inversion emulsions. The emulsions for use in the sense of the present invention are preferably compatible with the following galenic formulations: natural sprays, aerosols, gels, creams, lotions and/or sticks. Other useful galenic formulations for topical administration include, but are not limmited to, ointments, liposomes, liquid suspensions, foams, or mixtures thereof. The topical composition can also be disposed on a sheet to form a wet wipe product. The emulsion to be selected depends upon the desired formulation. For instance, both water-in-oil and oil-in-water emulsions are compatible with natural spray, aerosol, lotion, cream, gel and stick formulations, while w/o/w emulsions, GELTRAP emulsions and SWOP emulsions are compatible with natural spray, aerosol, lotion, cream or gel formulations but not with a stick formulation. Further, the formulations used are preferably water resistant.

Emulsions can be prepared according to methods well known in the state of the art. The appropriate carriers, and amounts thereof, can readily be determined by those skilled in the art according to the type of formulation being prepared. The components that constitute the base of the emulsion make up the "emulsifying system", to which the aqueous phase, the oil phase, active agents and further excipients are to be added. Non-limiting examples of emulsifying systems that are suitable for use in the present invention are disclosed in the examples below.

In one particular embodiment, the composition of the invention is a lotion that contains an emulsifying system that comprises polyglyceryl-4 diisostearate/polyhydroxystearate/sebacate, hydrogenated castor oil, and microcrystalline wax.

The compositions of the invention can contain one or more sunscreen agents. The sunscreen agents that can be used in the present invention must be capable of absorbing or blocking the harmful effects of ultraviolet radiation. In addition, they must be non-toxic and non-irritating when applied to the skin. The sunscreen agent can be included in the formulation at about 1 % to about 40 % (w/w) of the total composition. The amount of sunscreen agent in the composition will vary in the above range dependent upon the sun protection factor (SPF) desired. The higher the SPF, the greater the total amount of sunscreen agent. Preferably, the sunscreen agents are included at a concentration between 4 and 35 % (w/w) to achieve a SPF of 2 to 50+. More preferably, the sunscreen agents are included at a concentration between 10 and 30 %. Still more preferably, the sunscreen agents are included at a concentration between 19 and 23 % for high SPF products.

A wide selection of sunscreen agents which are appropriate for topical applications are well known in the state of the art. Non-limiting examples of sunscreen agents to be used in the compositions of the invention are octocrylene, butyl methoxydibenzoylmethane, bis-ethylhexyloxyphenol methoxyphenyl triazine, diethylhexyl butamido triazone, ethylhexyl triazone, and titanium dioxide, or mixtures thereof.

The compositions of the invention can incorporate other active ingredients. Non-limiting examples of adequate active agents for use in the compositions of this invention are: skin-soothing agents, antihistamines, anti-oxidants, and vitamins.

The topical compositions defined above comprise appropriate excipients for topical administration that can be pharmaceutical and/or cosmetical excipients, including, but not limited to, emulsifiers, protectives, adsorbents, emollients, surfactants, stabilizers, preservatives, moisturizers, buffering agents, solubilizing agents, dry-feel agents, waterproofing agents, antifoaming agents and skin-penetration agents. The excipients used have affinity for the skin, are well tolerated, stable and are used in an amount adequate to provide the desired consistency and ease of application. Additionally, the compositions may contain other ingredients, such as fragrances, colorants, and other components known in the state of the art for use in topical formulations.

The topical composition of the invention is for application on the skin of the subject who is susceptible of entering into contact with a cnidarian or a myxozoan organism to avoid the sting of these organisms. The composition is effective when applied prior to contact with these organisms. Preferably, the composition will be applied between 15 and 30 minutes before sun exposure or swimming, and will be reapplied frequently, specially after swimming.

Throughout the description and claims the word "comprise" and variations of the word, are not intended to exclude other technical features, additives, components, or steps. Unless otherwise defined, all technical and scientific terms used herein have the same meaning as commonly understood by one of ordinary skill in the art to which this invention belongs. Methods and materials similar or equivalent to those described herein can be used in the practice of the present invention.

Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### BRIEF DESCRIPTION OF THE DRAWINGS

FIG. 1. Interaction between Lanthanum and Zinc cations in the inhibition of cnidocyst discharge in Pelagia noctiluca. Zn²⁺ 0.233 % (A), Zn²⁺ 0.174 % and La³⁺ 0.03 % (B), Zn²⁺ 0.116 % and La³⁺ 0.06 % (C), Zn²⁺ 0.058 % and La³⁺ 0.09 % (D), La³⁺ 0.12 % (E). Number of discharged cnidocysts/mm² (Y). Large cnidocysts (L), small cnidocysts (S), total cnidocysts (T). Additivity line (α). Synergy curve (Ø).
FIG. 2. Interaction between Lanthanum and Zinc cations in the inhibition of cnidocyst discharge in Rhizostoma pulmo. Zn²⁺ 0.116 % (A), Zn²⁺ 0.058 % and La³⁺ 0.03 % (B), La³⁺ 0.06 % (C). Number of total discharged cnidocysts/mm² (Y). Additivity line (α). Synergy curve (Ø).

### DETAILED DESCRIPTION OF PARTICULAR EMBODIMENTS

### Example 1: emulsifying systems

The following emulsifying systems are useful when formulating the compositions of the invention.

**Table 1. Emulsifying systems for oil-in-water emulsion (o/w)**

| Non-ionic, anionic emulsions | |
|---|---|
| | % of total formulation (w/w) |
| Glyceryl stearate, PEG 100 stearate | 1.5 - 5 |
| Peg-40 stearate | 0.5-4 |
| Cetearyl alcohol | 0.5-4 |
| Sclerotium gum | 0.1-2 |
| Polyacrylamide, c13-14 isoparaffin | 0.1-2 |
| | |

| Cationic emulsions | |
|---|---|
| | % of total formulation (w/w) |
| Distearydimonium chloride | 2-6 |
| Dihydrogenated palmoylethyl hydroxyethylmonium methosulfate | 1-4 |
| Stearyl alcohol | 0-5 |
| Cetearyl alcohol | 0-5 |
| Glyceryl stearate | 0-3 |
| Polyacrylate-13 & polyisobutene & polysorbate 20 | 0.1-2 |

**Table 2. Emulsifying systems for water-in-oil emulsions (w/o)**

| | % of total formulation (w/w) |
|---|---|
| Polyglyceryl-4 isostearate | 1.5-5 |
| Microcristalline wax | 0.1-1 |
| Hydrogenated castor oil | 0.1-1 |

**Table 3. Emulsifying systems for multiple emulsions (w/o/w)**

| | % of total formulation (w/w) |
|---|---|
| Polyglyceryl-4 isostearate | 1.5-5 |
| Microcristalline wax | 0.1-1 |
| Hydrogenated castor oil | 0.1-1 |
| Acrylates (pemulen tr-2) | 0.05-2 |
| Polyacrylate-13 & polyisobutene & polysorbate 20 | 0.1-5 |

**Table 4. GELTRAP emulsions**

| | % of total formulation (w/w) |
|---|---|
| Octyldodecanol/octyldodecyl | 1.5-5 |
| Xyloside/peg-30 dipolyhydroxystearate | 1-6 |
| Polyacrylate-13 & polyisobutene & polysorbate 20 | 0.1-5 |

**Table 5. SWOP emulsions**

| Non-ionic, anionic emulsions | |
|---|---|
| | % of total formulation (w/w) |
| Polyglyceryl-4 isostearate | 1.5-5 |
| Decyl glucoside | 0.5-3 |
| Xanthan gum | 0.2-2 |
| Acrylamide/sodium acrylate copolymer; paraffinum | |
| liquidum; trideceth-6 | 0.5-2.5 |
| Myristyl myristate | 0.5-3 |

### Example 2: Interaction between zinc and lanthanum cations in the inhibition of cnidocyst discharge

Two substances that produce similar effects will sometimes produce exaggerated (synergism), diminished (antagonism) or similar (additivity) effects when used concurrently. A quantitative assessment is necessary to distinguish the synergism and the antagonism cases from a simply additive action.

The interaction between zinc and lanthanum cations was investigated by observing the effect of each of the single cations and their combinations on the inhibition of cnidocyst discharge. For the evaluation of cnidocyst discharge, a method based in physical contact of the cnidarian with a gelatin probe was employed. Said probes, besides stimulating the cnidocyte battery, retained the discharged cnidocysts, thereby allowing a quantitative evaluation of the response.

Gelatin probes were made coating one end of a 6 cm length of monofilament fishing line (1 mm diameter) with 25% (w/v) gelatin in deionized water and then placed in a humidified atmosphere during 30 minutes for gelatin solidification. ZnCl₂, LaCl₃ and combinations thereof were dissolved in deionized water at selected concentrations before the addition of gelatin. In preliminar experiments, the concentrations used for the individual constituents, i.e. of Zn²⁺ and La³⁺ when used on their own, had been found to be effective in inhibiting cnidocyst discharge.

Plain gelatin covered probes (no embebed active compound) were used as controls and represented maximum discharge figures.

After solidification, each gelatin probe was gently wiped across oral arms of Pelagia noctiluca and 5 times from 1 to 5 seconds in the tank. Pelagia noctiluca is one of the most widespread jellyfish species in the Mediterranean Sea. The probes were then placed in humidified atmosphere plates and observed under a steroemicroscope for cnidocyst quantification. Individual cnidocysts that had discharged into the gelatin probes were counted in 7 different fields of view and the mean value was calculated. Results are the average of triplicate probes.

Both Zn²⁺ and La³⁺ cations, as well as their combinations, exhibited an inhibitory effect, since they diminished the number of discharged nematocytes per mm² of gelatin probe as compared with controls. The obtained results, which are graphically represented in FIG. 1, show that the combinations of Zn²⁺ and La³⁺ cations are more effective that each component on its own, thus exhibiting a synergistic interaction.

Equivalent results were found for the effect of these agents on the inhibition of cnidocyst discharge in Rhizostoma pulmo, which is also one of the most common jellyfish species along the Mediterranean (see FIG. 2).

### REFERENCES CITED IN THE DESCRIPTION

- Salleo et al., J. exp. Biol., 1994, vol. 187, p. 201-206
- WO 98/53807
- WO 05/076731
- Boer Kimball A. et al, Wilderness and Environmental Medicine, 2004, vol. 15, p. 102-108

## Claims

1. Use of a combination of an effective amount of Zn²⁺ and La³⁺ cations for avoiding the sting of poisonous organisms that contain cnidocysts.

2. Use of the combination according to claim 1, wherein the weight ratio of Zn²⁺ to La³⁺ is comprised between 1:50 and 50:1.

3. Use according to any of the claims 1-2, wherein a zinc halide and a lanthanum halide are the sources which provide the Zn²⁺ and La³⁺ cations of the combination.

4. Use according to any of the claims 1-3, wherein the cnidocyst is from an organism selected from the group consisting of Cnidaria and Myxozoa.

5. Use of a topical composition comprising the combination as defined in any of the claims 1-4, together with appropriate topical pharmaceutically or cosmetically acceptable excipients and/or carriers, for avoiding the sting of poisonous organisms that contain cnidocysts.

6. Use according to claim 5, wherein the cnidocyst is from an organism selected from the group consisting of Cnidaria and Myxozoa.

7. Use according to any of the claims 5-6, wherein the effective amount of zinc cation is between 0.01 and 4% (w/w) and the effective amount of lanthanum cation is between 0.01 and 4% (w/w) of the total composition.

8. Use according to claim 7, wherein the effective amount of zinc cation is between 0.05 and 2.5% (w/w) and the effective amount of lanthanum cation is between 0.05 and 2.5% (w/w) of the total composition.

9. Use according to any of the claims 5-8, further comprising an emulsion system.

10. Use according to claim 9, wherein the emulsion system comprises polyglyceryl-4 diisostearate/polyhydroxystearate/sebacate, hydrogenated castor oil, and microcrystalline wax.

11. Use according to any of the claims 5-10, further comprising one or more cosmetical or pharmaceutical active agents selected from the group consisting of sunscreen agents, skin-soothing agents, antihistamines, antioxidants and vitamins.

12. Use according to any of the claims 5-11, wherein the sunscreen agent is selected from the group consisting of octocrylene, butyl methoxydibenzoylmethane, bis-ethylhexyloxyphenol methoxyphenyl triazine, diethylhexyl butamido triazone, ethylhexyl triazone, titanium dioxide, and mixtures thereof.

13. Use according to any of the claims 5-12, wherein the composition is administered in the form of a cream, a lotion, a natural spray, an aerosol, a gel, a foam, a suspension, a wipe, or a stick.

14. A method for avoiding the sting of an organism selected from the group consisting of Cnidaria and Myxozoa comprising the topical administration of the composition as defined in any of the claims 5-13, prior to contact with said organism.

## Patentansprüche

1. Verwendung einer Kombination von einer wirksamen Menge von Zn²⁺ und La³⁺ Kationen zur Verhinderung von Stichen von giftigen Organismen, die Cnidozysten beinhalten.

2. Verwendung der Kombination nach Anspruch 1, wobei das Gewichtsverhältnis von Zn²⁺ zu La³⁺ bei zwischen 1:50 und 50:1 liegt.

3. Verwendung nach einem der Ansprüche 1-2, wobei ein Zinkhalid und ein Lanthanumhalid die Sourcen sind, welche die Zn²⁺ und La³⁺ Kationen der Kombination bereitstellen.

4. Verwendung nach einem der Ansprüche 1-3, wobei die Cnidozyste aus einem Organismus ist, der aus der Gruppe bestehend aus Cnidaria und Myxozoa ausgewählt ist.

5. Verwendung einer topischen Zusammensetzung umfassend die Kombination wie in einem der Ansprüche 1-4 definiert nebst geeigneten topischen pharmazeutisch oder kosmetisch akzeptablen Hilfsstoffen und/oder Wirkstoffträgern zur Verhinderung des Stiches von giftigen Organismen, die Cnidozysten beinhalten.

6. Verwendung nach Anspruch 5, wobei die Cnidozyste aus einem Organismus ist, der aus der Gruppe bestehend aus Cnidaria und Myxozoa ausgewählt ist.

7. Verwendung nach einem der Ansprüche 5-6, wobei die wirksame Menge von Zink-Kation bei zwischen 0,01 und 4 Gew.-% und die wirksame Menge von Lanthanum-Kation bei zwischen 0,01 und 4 Gew.-% der gesamten Zusammensetzung liegt.

8. Verwendung nach Anspruch 7, wobei die wirksame Menge von Zink-Kation bei zwischen 0,05 und 2,5 Gew.-% und die wirksame Menge von Lanthanum-Kation bei zwischen 0,05 und 2,5 Gew.-% der gesamten Zusammensetzung liegt.

9. Verwendung nach einem der Ansprüche 5-8 weiterhin umfassend ein Emulsionssystem.

10. Verwendung nach Anspruch 9, wobei das Emulsionssystem Polyglyceryl-4 Diisostearat/Polyhydroxystearat/Sebacat, gehärtetem Rizinusöl und mikrokistallinem Wachs umfasst.

11. Verwendung nach einem der Ansprüche 5-10 weiterhin umfassend einen oder mehrere kosmetisch oder pharmazeutisch Wirkstoffe, die ausgewählt sind aus der Gruppe bestehend aus Sonnenschutzmitteln, hautberuhigenden Mitteln, Antihistaminika, Antioxidationsmitteln und Vitaminen.

12. Verwendung nach einem der Ansprüche 5-11, wobei das Sonnenschutzmittel ausgewählt ist aus der Gruppe bestehend aus Octocrylen, Butymethoxydibenzoilmethan, Bis-ethylhexyloxyphenol, Methoxyphenyltriazin, Diethylhexylbutamidotriazon, Ethylhexyltriazon, Titaniumdioxid und Mischungen davon.

13. Verwendung nach einem der Ansprüche 5-12, wobei die Zusammensetzung als Creme, Lotion, natürlicher Spray, Aerosol, Gel, Schaum, Aufschwemmung, Feuchttuch, oder Stift verabreicht wird.

14. Verfahren zur Verhinderung des Stiches von einem Organismus, der ausgewählt aus der Gruppe bestehend aus Cniadria und Myxozoa ist, umfassend die topische Verabreichung der Zusammensetzung wie in einem der Ansprüche 5-13 definiert, bevor es zum Kontakt mit dem Organismus kommt.

## Revendications

1. Utilisation d'une combinaison d'une quantité efficace de cations Zn²⁺ et La³⁺ pour éviter la piqûre d'organismes venimeux contenant des cnidocystes.

2. Utilisation de la combinaison selon la revendication 1, dans laquelle le rapport en poids de Zn²⁺ à La ³⁺ est compris entre 1:50 et 50:1.

3. Utilisation selon l'une quelconque des revendications 1-2, dans laquelle un halogenure de zinc et un halogenure de lanthanum sont les sources qui fournissent les cations Zn²⁺ et La³⁺ de la combinaison.

4. Utilisation selon l'une quelconque des revendications 1-3, dans laquelle le cnidocyste est d'un organisme choisi dans le groupe constitué de Cnidaria et Myxozoa.

5. Utilisation d'une composition topique comprenant la combinaison telle que définie dans l'une quelconque des revendications 1-4 conjointement avec des excipients et/ou des véhicules topiques pharmaceutiquement ou cosmétiquement acceptables appropriés pour éviter la piqûre d'organismes venimeux contenant des cnydocystes.

6. Utilisation selon la revendication 5, dans laquelle le cnidocyste est d'un organisme choisi dans le groupe constitué de Cnidaria et Myxozoa.

7. Utilisation selon l'une quelconque des revendications 5-6, dans laquelle la quantité efficace de cation zinc est comprise entre 0,01 et 4% en poids et la quantité efficace de cation lanthanum est comprise entre 0,01 et 4% en poids par rapport a la composition totale.

8. Utilisation selon la revendication 7, dans laquelle la quantité efficace de cation zinc est comprise entre 0,05 et 2,5% en poids et la quantité efficace de cation lanthanum est comprise entre 0,05 et 2,5% en poids par rapport à la composition totale.

9. Utilisation selon l'une quelconque des revendications 5-8 comprenant en outre un système d'émulsion.

10. Utilisation selon la revendication 9, dans laquelle le système d'émulsion comprend du polyglycéryl-4 diisostéarate/polyhydroxystéarate/sébacate, de l'huile de ricine hydrogéné, et de la cire microcristalline.

11. Utilisation selon l'une quelconque des revendications 5-10 comprenant en outre un ou plusieurs agents actifs cosmétiques ou pharmaceutiques choisis dans le groupe constitué des écrans solaires, des agents apaisants de la peau, des antihistaminiques, des antioxydants et des vitamines.

12. Utilisation selon l'une quelconque des revendications 5-11, dans laquelle l'écran solaire est choisi dans le groupe constitué de l'octocrylène, du butyl méthoxydibenzoyl-méthane, de la bis-éthylhéxyloxyphénol méthoxyphényl triazine, de la diéthylhéxyl butamido triazone, de la éthylhéxyl triazone, du dioxyde de titanium, et des mélanges de ceux-ci.

13. Utilisation selon l'une quelconque des revendications 5-12, dans laquelle la composition est administrée sous la forme d'une crème, d'une lotion, d'un spray naturel, d'un aérosol, d'un gel, d'une mousse, d'une suspension, d'une lingette, ou d'un stick.

14. Méthode pour éviter la piqûre d'un organisme choisi dans le groupe constitué de Cnidaria et Myxozoa comprenant l'administration topique d'une composition telle que définie dans l'une quelconque des revendications 5-13 avant le contact avec ledit organisme.
